# EUROPEAN PATENT SPECIFICATION

(11) **EP 1 797 423 B1**
(45) Date of publication and mention of the grant of the patent: **22.02.2017**
(21) Application number: 05796893.5
(22) Date of filing: 15.09.2005
(51) Int. Cl.: G01N 33/28, G01N 25/08

(54) **METHOD OF ASSAYING A HYDROCARBON-CONTAINING FEEDSTOCK**
VERFAHREN ZUM PRÜFEN EINES KOHLENWASSERSTOFFE-ENTHALTENDEN ROHMATERIALS
PROCÉDÉ D'ANALYSE D'UNE CHARGE D'ALIMENTATION CONTENANT UN HYDROCARBURE

(30) Priority: 17.09.2004 US 611050 P; 17.09.2004 US 611002 P
(43) Date of publication of application: 20.06.2007
(73) Proprietor: BP OIL INTERNATIONAL LIMITED, Sunbury-on-Thames TW16 7BP (GB)
(72) Inventor: VOELKENING, Joachim, Gelseukischen 45897 (DE); HODGES, Michael, Wonersh, Surrey GU5 05A (GB)
(74) Representative: Hamer, Christopher K.
(86) International application number: PCT/US2005/033247
(87) International publication number: WO 2006/034072

(56) References cited:
- WO-A-03/048759
- US-A- 3 169 389
- US-A- 3 996 786
- US-A- 4 971 915
- US-A- 5 699 269
- SEINSCHE K ET AL: "ANWENDUNG DER PLS-REGRESSIONSMETHODE AUF DATEN DER GASCHROMATOGRAPHIE UND DER IR-SPEKTROSKOPIE. QUALITY CONTROL OF JET FUELS - PLS REGRESSION ANALYSIS OF GASCHROMATOGRAPHIC AND IR-SPECTROSCOPIC DATA" ERDOEL ERDGAS KOHLE, URBAN VERLAG, HAMBURG, DE, vol. 112, no. 6, June 1996 (1996-06), pages 261-263, XP000641535 ISSN: 0179-3187

## Description

### BACKGROUND OF THE INVENTION

### Field of the Disclosure

The disclosure generally relates to a method of obtaining a determinative assay of a hydrocarbon-containing feedstock such as crude oils, syncrudes, refining intermediates, and bio components from limited information of the feedstock.

### Brief Description of Related Technology

A hydrocarbon-containing feedstock such as crude oil is a complex mixture of hydrocarbons and heteroatomic organic compounds having varying molecular weights and polarity. Crude oil seldom is used in the form obtained from a well; instead, it is typically converted into a wide range of usable fuels by a combination of physical and chemical processes, collectively known as refining. Crude oils vary widely in their refining characteristics and commercial value. Sales negotiations and chemical processing involving hydrocarbon-containing feedstocks such as refinery feedstocks can benefit from timely and accurate information concerning the chemical composition and physical and performance properties of crude oil. Oftentimes, however, such information is simply unavailable at the time of the purchase and, therefore, a trader negotiating the purchase of a refinery feedstock must make a purchase decision on the bases of historical information concerning the feedstock's source and an expectation that the feedstock will possess substantially the same characteristics as those purchased in the past from the same source (or similar region of the world). With more timely information about the feedstock, however, the trader gains the benefit of knowing whether the feedstock under sales consideration is better or worse than the product that he normally would expect to obtain from the source and, thus, can better negotiate a price based on the actual chemical composition and physical and performance properties of the particular product instead of on the bases of historical information and expectations. Furthermore, to the extent that there might be unexpected constituents present in the feedstock - constituents that might, for example, damage a refinery process or are otherwise undesirable - the trader benefits from full and accurate information about the feedstock at the time of the sales negotiations. Heretofore, however, it has been impractical to obtain timely and accurate information at the time of the negotiation and potential sale because numerous properties of the refinery feedstock must be determined and conveyed to the trader. Traditionally, such analyses require a large sample volume of the feedstock and take one to two weeks to complete. Thus, it is customary for a refinery feedstock to be purchased by a trader having incomplete (or indeterminative) assay information concerning the purchased feedstock.

Similarly, a refinery feedstock assay such as crude oil assay is an important analysis that must be performed before crude oils are refined. Typically, an oil refinery will refine a large number of different crude oils (and blends thereof), each of which may differ in a number of important physical and performance properties, to obtain a particular distillate cut for further manufacture or sales. Again, numerous properties of the refinery feedstock must be analyzed before refinery engineers can determine the optimum refinery process conditions for refining each feedstock and evaluate the potential effects each feedstock may have on the refinery equipment (e.g., corrosion, deposition, etc.). These engineers determine the particular blending ratios and other processing steps based on historical data profiling the constituent components and properties of each crude oil based on their source. The constituent components, however, and the overall properties of the crude oil can change over the course of time and during transport from the source to the refinery. Thus, when the refinery feedstock arrives at the refinery it may often possess characteristics different from those determined when the crude oil was being produced from the source. These differences can dramatically affect the blending ratios necessary to obtain a desired distillate cut. Having purchased a refinery feedstock based on incomplete information, necessary analyses must be performed prior to refining to obtain determinative assay information. As noted above, these analyses require a large sample volume of the product and take one to two weeks to complete. If, after having obtained the results of the analyses, the refinery feedstock does not possess the expected chemical composition and physical and performance properties, the refinery engineers must modify the process accordingly, thus, resulting in additional inefficiencies.

Commercially-available software products, such as, for example, products from Spiral Software Ltd. (United Kingdom), are capable of providing determinative assay data for a refinery feedstock based on limited information of the feedstock (e.g., true boiling profile, sulfur-content, nitrogen-content, density, product yields, etc.) and a comprehensive database containing complete (or determinative) assays of hundreds (preferably, thousands) of known crude oils, syncrudes, refining intermediates, bio-components. To make proper use of such software products, the database must be of a high quality and have information on a variety of crude oils and other refinery feedstocks based on type and source. These software products can take the limited information and perform complex mathematical operations to match the feedstock (based on the limited known information) to the assay of a known crude oil having the most similar attributes. Alternatively, these software products can take the limited information and perform complex mathematical operations to construct or reconstruct a determinative assay of an unknown crude oil, syncrude, intermediate etc., based on the interrelationships of the various properties and on the variety of known crude oils, syncrudes, refining intermediates, etc present in the database. These software products, thus, require an input of limited assay information obtained from a sample volume of a refinery feedstock, and a comprehensive database. Independent of the usefulness of the software products are the speed and accuracy of the tests run to obtain the limited assay information and also the value of the limited assay information in accurately predicting the determinative assay information of the refinery feedstock.

Whether the refinery feedstock is being evaluated at the point of sale or has just arrived at an oil refinery, there is a need to have as complete (or determinative) assay information as soon as possible to make meaningful purchasing and processing decisions. Complete or determinative assay information can include information concerning the chemical composition and physical properties of the refinery feedstock and performance properties thereof. Based on known technology, however, quick and determinative assay information of a refinery feedstock is difficult to obtain. While software exists to predict determinative assay information based on limited assay data, labor- and time-intensive processing and analyses are required to obtain the limited assay data necessary to use the software. Specifically, just to obtain the limited data necessary to use the software, one needs to distill the crude oil into multiple fractions and then analyze each fraction to obtain data for numerous physical properties. Even the more recent advances in the art such as, for example, those disclosed in International Publication No. WO 00/39561 A1, teach methods of automatically analyzing crude oils with spectroscopy, but require significant-size quantities of the crude oil and conventional distillation equipment. Moreover, such methods still require about two days to complete. Other advances in the art, such as, for example, those disclosed in International Publication No. WO 03/048759 A1, require obtaining assay data on hundreds of parameters before a determinative assay can be generated. Thus, the methods do not beneficially assist the trader that is negotiating the purchase of a refinery feedstock at its source or the refinery engineer when the refinery feedstock arrives at the refinery.

It would be desirable to develop tests capable of quickly obtaining limited assay information. Furthermore, it also would be desirable to determine which properties of a hydrocarbon-containing feedstock are especially predictive of an accurate, determinative assay of the feedstock. Based on such a determination, in accordance with the method disclosed herein only those tests that are capable of providing results especially predictive of a determinative assay need to be performed.

### SUMMARY OF THE INVENTION

Disclosed herein is a method of assaying hydrocarbon-containing feedstocks such as refinery feedstocks including but not limited to crudes, synthetic crude-oils, partially refined intermediate fractions such as a residue component or a cracked stock component, bio-components or blends thereof, and petroleum exploration pre-production test well samples, according to claim 1.

The techniques are selected from the group consisting of ultraviolet-visible (UV-Vis) absorbance spectroscopy, infrared (IR) absorbance spectroscopy, UV fluorescence spectroscopy, mid-infrared (MIR) absorbance spectroscopy, near infrared (NIR) absorbance spectroscopy, X-ray fluorescence (XRF) spectroscopy, nuclear magnetic resonance, micro-oscillation, micro-distillation, micro-mass spectrometry, micro-ion mobility spectrometry, and micro-gas chromatography.

Additional features of the disclosure may become apparent to those skilled in the art from a review of the following detailed description and the appended claims.

### DETAILED DESCRIPTION OF THE INVENTION

The present invention relates to a method of assaying a hydrocarbon-containing feedstock comprising measuring boiling profile and at least one other property with at least two different laboratory-independent measuring techniques wherein one technique is predictive of the boiling profile and the other technique is predictive of the other property, transmitting the measurements to a processor capable of reconstructing a determinative assay of the hydrocarbon-containing feedstock from the measurements, and reconstructing a determinative assay of the hydrocarbon-containing feedstock from the measurements, wherein the other property is selected from the group consisting of density, specific gravity, total acidic number, pour point, viscosity, sulfur content, metal content, nitrogen content, and combinations thereof. Preferably the boiling profile is the true boiling profile.

The measuring step preferably includes performing at least two techniques selected from the group consisting of ultraviolet-visible (UV-Vis) absorbance spectroscopy, infrared (IR) absorbance spectroscopy, UV fluorescence spectroscopy, mid-infrared (MIR) absorbance spectroscopy, near infrared (NIR) absorbance spectroscopy, X-ray fluorescence (XRF) spectroscopy, nuclear magnetic resonance, micro-oscillation, micro-distillation, micro-mass spectrometry, micro-ion mobility spectrometry, and micro-gas chromatography (GC). In each case, the measuring technique selected in accordance with the method of assay is the technique most predictive of the property in question. By making such a selection, the method of assay disclosed herein permits the reconstruction of the hydrocarbon sample by measuring only a limited number of key properties, i.e., at least two properties.

Preferably, the method includes performing at least two of the aforementioned techniques with a laboratory-independent device, correlating data obtained from the techniques to obtain boiling profile and at least one other property of the crude oil, transmitting the boiling profile and other property to a processor capable of reconstructing a determinative assay of the crude oil from the measurements, and reconstructing a determinative assay of the crude oil.

In a preferred embodiment, the measuring technique includes measuring the boiling profile of the sample with at least one of mid-infrared (MIR) absorbance spectroscopy, near infrared (NIR) absorbance spectroscopy, nuclear magnetic resonance, micro-oscillation, micro-distillation, and micro-gas chromatography (GC). The spectral data obtained via these spectroscopic techniques or the data obtained by the nuclear magnetic resonance, micro-oscillation, micro-distillation, and micro-gas chromatography (GC) techniques can be correlated to the boiling profile of the sample using chemometric analyses known by those having ordinary skill in the art. These techniques can be performed on the crude oil sample itself or, alternatively, on distilled fractions of the crude oil sample. For example, the sample may be subject to separation into various fractions by micro-gas chromatography (GC) or micro-distillation and, subsequently, each fraction can be irradiated with mid-infrared (MIR) and/or near infrared (NIR) light. The spectral data obtained from the irradiation can be correlated using chemometric analyses to the boiling profile of a reference material whose spectral data are stored in a database, for example.

In another preferred embodiment, the other property is selected from the group consisting of density, specific gravity, total acidic number, pour point, viscosity, and combinations thereof, and the measuring step includes measuring the property by performing at least one technique selected from the group consisting of ultraviolet-visible (UV-Vis) absorbance spectroscopy, infrared (IR) absorbance spectroscopy, UV fluorescence spectroscopy, mid-infrared (MIR) absorbance spectroscopy, near infrared (NIR) absorbance spectroscopy, X-ray fluorescence (XRF) spectroscopy, nuclear magnetic resonance, micro-oscillation, micro-distillation, and micro-gas chromatography (GC). Preferably, the technique is at least one of mid-infrared (MIR) absorbance spectroscopy, near infrared (NIR) absorbance spectroscopy, and nuclear magnetic resonance.

In still another preferred embodiment, the other property is selected from the group consisting of sulfur content and metal content (e.g., nickel content, vanadium content, iron content, etc.) and combinations thereof, and the measuring step includes measuring the property with X-ray fluorescence (XRF) spectroscopy. In another preferred embodiment, the property is metal content (e.g., nickel content, vanadium content, iron content, etc.), and the measuring step includes measuring the property with ultraviolet-visible (UV-Vis) absorbance spectroscopy.

As noted above, the assaying methods disclosed herein generally include obtaining at least two properties, the boiling profile and at least one other property of the hydrocarbon-containing feedstock such as crude oil. This is accomplished by carrying out at least two aforementioned measuring techniques on the crude oil sample to obtain data wherein the measuring technique is always selected to determine the property it is most predictive of, and, thereafter, correlating the obtained data to the boiling profile and the other property using chemometric analyses known by those having ordinary skill in the art. The correlating step includes identifying the obtained spectrum, for example, and determining which property of the crude oil can be predicted by the spectrum. The correlating step also can include identifying the obtained data and determining which property of the crude oil can be predicted by the data. The correlating step also preferably includes matching the obtained spectrum or data to the property of a reference material whose known spectrum or data are stored in a database, or using chemometric analyses to determine the property of the sample.

For example, to correlate the density of a crude oil to an NIR spectrum, one should select a calibration set of samples covering the whole range of the property that is intended to be measured in the future. A correlation for density might include the density of samples evenly distributed between 700 kilograms per cubic meter (kg/m³) and 1100 kg/m³. From those samples, the NIR spectra and the density can be measured using conventional ASTM methodology (e.g., ASTM 1655-00 for NIR, and IP 365 for density) or other well-known standard methodologies. Using chemometric analyses, such as, for example, multilinear correlation algorithms like partial least square, multiple linear regression, neural networks, or genetic algorithms, a mathematical correlation between the NIR spectrum and the density can be determined. This mathematical correlation then can be used to derive the density of unknown samples from their NIR spectra. Specific gravity (API) of the sample can be calculated from the density. Similar calibrations can be performed for other properties of crude oil based on well-known standards as set forth in the following Table 1:

**Table 1**

| **Property** | **Standard** |
|---|---|
| Density | IP 365 |
| True Boiling Profile | ASTM D2892 |
| | ASTM D5236 |
| Nickel Content | IP 437 |
| Sulphur Content | IP 477 |
| Vanadium Content | IP 437 |
| Nitrogen Content | IP 379 |
| Acidity | ASTM D664 |
| Pour Point | ASTM D97 |
| Viscosity | ASTM D445 |

As noted above, the properties to be measured or correlated in accordance with the disclosed methods are selected from the group consisting of density, specific gravity, boiling profile, total acidic number, total base number pour point, viscosity, sulfur content, nickel content, vanadium content, and nitrogen content. Additional properties can be measured, however, it has been found that information on the selected properties is all that should be necessary to obtain a determinative assay of the hydrocarbon-containing feedstock under consideration. Chemical properties of a crude oil can include, but are not limited to, elemental and molecular compositions. Physical properties of a crude oil can include, but are not limited to, density, viscosity, yield structure, smoke point and cold flow properties such as pour point, cloud point, or freeze point. Performance properties of a hydrocarbon-containing feedstock can include, but are not limited to, octane number and cetane number. Other chemical, physical, and performance properties of crude oil are generally known by those having ordinary skill in the field of crude oil refining.

The assay method of the present invention is able to reconstruct the hydrocarbon-containing feedstock from data on limited key properties in part by using the optimum independent measuring technique most predictive of the property in question. In this connection for instance, one could use the NIR technique for determining boiling profile and density; the XRF technique for determining nickel, vanadium, or sulphur content; the micro rheological technique for determining viscosity and the conductivity technique for determining acidity.

The disclosed method uses a database containing a source of up-to-date and complete (or determinative) hydrocarbon-containing feedstock assay data for reference such as refinery feedstocks including crude oils, syncrudes, intermediates, and bio-components whose composition and physical and performance properties have been previously measured and characterized. A "determinative" crude oil assay is intended to refer to as complete information as might be desirably necessary for a trader or engineer to make a business or processing decision. Correlational data relative to these reference crude oils also can exist in the database. Such correlational data can include, for example, spectral data, and chemical, physical, and performance property data. A processor capable of accessing the database and containing software utilizing advanced mathematical methods can generate determinative assay information for hydrocarbon-containing feedstocks such as crude oils, including accurate re-cutting and flexible data exchange with other applications. Advanced statistical methods that are preferably a part of the software can be utilized by the processor to identify links between the correlational data and a particular property, building a crude oil model which can be used to reconstruct complete characteristics from available information. Even when based on only a few key measured parameters, these reconstructions span the entire range of physical and chemical properties. Error estimates on all predicted values allow a user (e.g., the trader, the refinery engineer, etc.) to assess any risk in associated business and processing decisions. These advanced statistical methods coupled with the limited assay information specified herein make possible the reconstruction of a determinative hydrocarbon-containing feedstock from limited measurements with remarkable accuracy. Accordingly, when an unknown hydrocarbon-containing feedstock is evaluated using at least two measuring techniques described herein, the measured data can be correlated with the data existing for one or more known references to generate a determinative assay of the unknown hydrocarbon-containing feedstock. Suitable software products are commercially available from, for example, Spiral Software Ltd. (United Kingdom). Suitable processors include, but are not limited to, both general and special purpose microprocessors, such as those typically found in industrial computers, personal computers, and personal digital assistant (PDA) devices.

Any multivariate technique may be used as a correlational technique (i.e., to correlate the measured data with the composition and physical and performance property data existing for the known reference). A multivariate measurement is one in which multiple measurements are made on a sample of interest - in other words, more than one variable or response is measured for each sample. Thus, for example, using a sensor array to obtain multiple responses on a vapor sample is a multivariate measurement. See generally, Beebe et al. "Chemometrics: A Practical Guide," 6 (John Wiley & Sons Inc. 1998). The preferred correlational techniques are sparse data techniques, chemometric techniques such as, for example, partial least square, multiple linear regression, genetic algorithms, and neural networks. Thus, the data obtained with multivariate techniques is mathematically correlated with an intrinsic property of the composition to produce information about the property.

For example, NIR spectra can be used to measure the intensity of the overtones of the molecular vibrations in a molecule, like carbon-hydrogen, oxygen-hydrogen, and nitrogen-hydrogen bonds and the molecule. The carbon-hydrogen (C-H) absorption bands are typically useful for mixtures of organic compounds. Different types of C-H bonds, e.g., aromatic, aliphatic, and olefinic hydrocarbons, absorb light at different characteristic frequencies. The magnitude of the absorption band is proportional to the amounts of the C-H bonds in the sample. Therefore the NIR spectrum can provide a fingerprint of the sample composition. This fingerprint can be empirically correlated to the intrinsic properties of the sample.

NIR spectroscopy has certain advantages over other analytical methods in refineries and can cover a large number of repetitive applications accurately and quickly. The NIR region between 800 nanometers (nm) and 2500 nm contains the totality of molecular information in the form of combinations and overtones from polyatomic vibrations, but mathematical techniques are needed to utilize this information and to calculate the desired parameters. U.S. Patent Nos. 5,490,085; 5,452,232; and 5,475,612, describe the use of NIR for determining octane number, yields and/or properties of a product of a chemical process or separation process from analysis on the feeds to that process, and yields and/or properties of a product of a blending operation again from analysis on the feed thereto.

When light strikes a fluid, several phenomena may occur. For example, a portion of the light may be reflected from the surface, while another portion will pass into the fluid. The portion of the light passing into the fluid may be transmitted through the fluid or subjected to scattering or absorption. Oftentimes, all of these mechanisms will occur simultaneously. The amount of light absorbed at a given wavelength is a characteristic of the substance through which the light travels. While the light that is absorbed cannot be directly measured, the light emerging from the fluid can be measured. As a result of absorption, the intensity of the emerging light will be reduced or "attenuated." The amount that light may be attenuated for any given composition will vary as a function of its wavelength. Thus, for a given source light spectrum, evaluating the intensity of the components of the emerging light at selected wavelengths provides information about the composition of the fluid.

Scattering also causes attenuation of the light intensity. However, whereas attenuation as a result of absorption causes relative changes in the light intensity as a function of wavelength, i.e., there is a change in the shape of the broadband spectrum, attenuation due to the scattering of light is much less dependent on its absolute wavelength; it has a slow, monotonic dependence on wavelength. The scattering of the light, therefore, results in a drop in the light intensity at all wavelengths so that at any given wavelength, the intensity does not change appreciably relative to the intensity at other wavelengths. For fluids which both scatter and absorb light, the net result is that even though the absolute magnitude of the collected light as a function of wavelength it is not uniquely related to chemical composition, the relative light intensity as a function of wavelength is related to the chemical composition. Because different wavelengths of light may behave differently, multiple measuring techniques are preferred according to the assay methods disclosed herein.

The methods disclosed herein are performed using one or more laboratory-independent devices. As used herein to modify the term "devices," the term "laboratory-independent" refers to devices that are portable and, preferably, hand-held. Furthermore such devices should be capable of being operated by one person. Thus, preferably, each device has a total weight of less than about five kilograms and more preferably less than about two kilograms. One or more laboratory-independent devices suitable for carrying out the measuring step can include one or more of a micro-distillation unit, a micro-oscillator unit, a micro-gas chromatograph (e.g., a micro-2-dimensional gas chromatograph, a micro-ultraviolet-visible (UV-Vis) light spectrometer, a micro-infrared (IR) spectrometer, a micro-UV fluorescence spectrometer, a micro-mid-infrared (MIR) spectrometer, a micro-near infrared (NIR) spectrometer, a portable X-ray fluorescence (XRF) spectrometer, micro-mass spectrometer, micro-ion mobility spectrometer, a portable nuclear magnetic resonance spectrometer, micro conductivity/capacitance devices, micro rheological devices and tuning-fork sensors. A suitable laboratory-independent device also can be multifunctional in that a single device is capable of performing one or more of ultraviolet-visible (UV-Vis) absorbance spectroscopy, infrared (IR) absorbance spectroscopy, UV fluorescence spectroscopy, mid-infrared (MIR) absorbance spectroscopy, near infrared (NIR) absorbance spectroscopy, X-ray fluorescence (XRF) spectroscopy, nuclear magnetic resonance, micro-oscillation, micro-distillation, micro-mass spectrometry, micro-ion mobility spectrometry, and micro-gas chromatography (GC).

Suitable spectrometers are commercially-available and known by those having ordinary skill in the field of crude oil refining. For example, micro-NIR spectrometers are commercially-available from Axsun Technologies Inc. (Massachusetts, USA), under the name AXSUN NIR-APS Analyzer. Micro-gas chromatographs are commercially-available from Siemens (under the name MicroSAM) and SLS (under the name Micro-technology). XRF spectrometers are commercially available from Oxford Instruments (United Kingdom). Suitable micro-oscillator units are generally described in U.S. Patent No. 5,827,952. Suitable tuning fork sensors are generally described in U.S. Patent No. 6,393,895.

While any one of these units or spectrometers is capable of providing data that can be correlated with multiple properties, it has been found that, oftentimes, certain parameters might be better detected by one unit or spectrometer over another. For example, in one preferred embodiment, the property to be detected is selected from the group consisting of density, specific gravity, total acidic number, pour point, and viscosity, and the irradiating step includes irradiating the sample hydrocarbon-containing feedstock with at least one of near infrared (NIR) light and mid-infrared (MIR) light. Thus, preferably, the laboratory-independent device includes more than one of the aforementioned units or spectrometers (e.g., the device is multi-functional) such that different units or spectrometers are utilized to obtain data on different properties.

As mentioned above, certain measuring techniques are more accurately predictive of certain properties than are other measuring techniques. The assay methods disclosed herein includes selecting a measuring technique that is predictive of the property sought to be measured. For example, a method can include selecting measuring technique predictive of the boiling point and another measuring technique predictive of another property of the hydrocarbon-containing feedstock, such as density, specific gravity, total acidic number, pour point, viscosity, sulfur content, metal content, and nitrogen content. As used herein the term "predictive" is meant generally to encompass techniques whose measurements fall within an acceptable range of error when compared to standardized methods (e.g., ASTM) of measuring the same property. An acceptable range of error may be within 15 percent, preferably within 10 percent, and more preferably 5 percent or less of the value measured by standardized methods (e.g., ASTM). Thus, where one measuring technique has a range of error in excess of 15 percent of a standardized method, such a technique would not be considered to be predictive of the property sought to be measured. In contrast, where another measuring technique has a range of error of 15 percent or less, then such a technique would be considered to be predictive of the property sought to be measured. Criteria for selecting a measuring technique include, but are not limited to, high precision (as discussed above), the speed at which the technique can be performed (fast techniques being preferred), the relative expense in performing the technique (inexpensive techniques being preferred), the ease of performing the technique (techniques not requiring skilled technicians being preferred), and a lack of sensitivity of a technique to changes in ambient conditions.

Preferably, each laboratory-independent device has a weight of about five kilograms or less, and more preferably about two kilograms or less. Furthermore, preferably, each laboratory-independent device requires only a small-size quantity of crude oil to perform the disclosed method. Accordingly, each laboratory-independent device preferably requires about 100 milliliters or less of a crude oil sample, more preferably about 10 mL or less, and most preferably 1 mL or less of the sample. With such small-size quantity requirements, and small size and weight of each laboratory-independent device, the devices can be made portable and taken to the location of the yet-to-be assayed crude oil - such as, for example, to the crude oil well or to the off-loading vessel. Advantageously, because of the relatively small size of the components of the laboratory-independent device, the power requirements also should be relatively low. Thus, the device can be operated with a suitable battery such as, for example, a rechargeable battery, without detrimentally adding weight to the over all device.

Typically, the device or devices used to carry out the present method provides an analysis in less than two hours, and preferably provides an analysis in less than 30 minutes, such as in less than 2 minutes.

Where the portable device or devices are used to carry out the method disclosed herein for analysis of a product of a refinery process, the product may be an intermediate stream in the overall refinery process, a bitumen, a product from the overall refinery process which is subsequently used as a chemical feedstock, or a product from the overall refinery process which is subsequently used as a blending component for a fuel or lubricant.

The analytical devices present in the portable apparatus can suitably be microfabricated, and may be in the form of sensors. Microfabricated devices are devices in which the crucial analytical part or detector of the device is fabricated using techniques consistent with the microchip industry, and produces a spectrum or a simple electrical signal, in response to contact with a test substance. A simple electrical signal can be fed to an associated set of electronics which either converts the input signal into a value for the property being measured, or further processes the signal using chemometric techniques. A spectrum may be used directly or mathematically treated before being subjected to chemometric techniques to yield the required property or properties. In either case, the value or spectrum can be fed to a model generated from the relationship between values or spectra measured and the known composition or properties of such samples determined by previous analytical measurements.

Where present, the micro-distillation or micro-fractionation device may be any suitable device which can be utilized to distill or fractionate a sample to give fractions similar to those achieved by conventional distillation. For example, the micro-distillation or micro-fractionation device may distil or fractionate a crude oil or other refinery feedstock to give fractions similar to those achieved by conventional refinery distillation in a crude distillation unit (CDU). The micro-distillation device may also be a micro engineered device comprising a micro-heater for vaporizing the sample (e.g. crude oil), a suitable channel, for example a capillary, through which the vaporized sample passes to achieve a vapor liquid separation, a suitable condensing zone (typically a cooled zone, such as a micro-refrigerator) on which vaporized sample that has passed up the channel condenses, and a micro-sensor to measure the condensation of sample at the condensing zone. The micro-sensor may be an optical sensor. Preferably, the micro-distillation device is a micro-fabricated separation device, for example, on a silicon wafer. The micro-distillation device may be disposable. Where the micro-distillation device provides a series of fractions similar to those achieved by conventional distillation, then these fractions can be analyzed by one or more further analysis devices.

The micro-oscillator device, when present, is preferably an acoustic optical device or sensor. Micro-oscillator devices are based on measurement of the frequency of oscillation of the device, which changes with mass of material on the oscillator. Thus, if material evaporates or condenses on the device, the frequency changes. As well as information on TBP, acoustic optical devices may provide information on viscosity, cold flow properties, volatile contaminants and deposits formation. Suitable micro-oscillators are described in U.S. Patent Nos. 5,661,233 and 5,827,952.

Micro-NIR, when present, may be used, for example, to provide information on TBP and to give a simulated distillation curve, as well as to provide information on density and amounts of saturates and aromatics in the sample as a whole and/or in fractions obtained from a suitable separation step, such as a micro-distillation device. Sulphur and/or cold flow properties, such as cloud point and freezing point, acidity (TAN), Research Octane Number (RON), Motor Octane Number (MON), cetane number and smoke point may also be measured. Suitable micro-NIR analyzers include the Axsun NIR-APS Analyser produced by Axsun Technologies Inc., Massachusetts.

The device for measuring density can be an oscillating sensor, and the device for measuring TAN can be an electrochemical sensor.

Micro-GC, when present, may provide a simulated distillation curve and can provide hydrocarbon speciation, such as of C1-C9 hydrocarbons. Suitable micro-GC devices include Siemens MicroSAM process GC's or SLS Micro-technology GC's.

Micro-ion mobility/differential mobility spectrometry, when present, may be used to provide information on specific molecular types and particularly on polar molecules in the sample, for example contaminants such as organic chlorides or methanol, as well as sulphides and nitrogen compounds. Further, micro-ion mobility/differential mobility spectrometry coupled with a micro pyrolyser, can give enhanced nitrogen and sulphur analysis. Micro-ion mobility/differential mobility spectrometry is best implemented in combination with micro GC and/or pre-fractionation/pre-concentration device. Suitable micro- ion mobility/differential mobility spectrometers include the Sionex microDMx.

For use in the present method a number of devices may be arranged in a single portable apparatus. The apparatus may include at least 3 different analysis devices, preferably at least 5 different analysis devices, such as at least 10 different analysis devices, allowing a number of properties of a sample (or of fractions thereof) to be ascertained using the apparatus, and providing a significant amount of data for the analysis, either directly or via a suitable database model as described further below.

Due to its portability, the apparatus used to carry out the method can be taken to the location of the sample to be analysed, and a rapid analysis of the sample obtained. For example, for crude oil analysis (assay), the apparatus may be used for "at location" rapid assessment/valuation of crude oils, for example on a crude oil tanker or in a land-based crude oil storage tank, or at an oil exploration drilling site, allowing the value of the crude oil to a potential purchaser to be quickly ascertained. At an oil exploration drilling site, the apparatus may be used at the "well-head" on the drilling site to provide rapid analysis of a crude oil, for example, to provide rapid feedback of the properties of a crude oil at a test well allowing evaluation of said crude oil.

Preferably the apparatus that can be used with the present assay method is at least compatible with, wireless communications, such as a wireless mesh network, and more preferably, with remote communications means, such as satellite-based data communication, such that the analysis results may be readily communicated to the potential purchaser, again reducing the time-scale on which the analysis data is available to the potential purchaser.

Especially where suitable micro-devices are not available, the apparatus that can be used to carry out the present method may be used in combination with other portable analysers, particularly those yielding elemental data, such as portable X-Ray Fluorescence (XRF) spectroscopy and Laser Induced Breakdown Spectroscopy (LIBS) to improve the breadth of assay.

XRF, for example, can provide analysis of sulphur and metals content of a sample, for example of crude oil fractions. Suitable, portable, XRF analysers include those available from OXFORD instruments.

Generally, the apparatus used to carry out the assay method of the present invention, optionally in combination with any other analysers, will generate data in at least two and preferably in respect of at least 10 key properties of the sample to be analyzed.

Because of the rapid analysis obtainable by using the assay method of the present invention, analyses can be obtained more often and/or can be used for process optimisation. For example, the method may be used at a refinery and regular analyses can be performed on blends of refinery feedstocks, such as blends of crude oils, produced (from two or more sources available) at the refinery, to ensure optimum configuration of the refinery for the blend. Further the method may be used to verify consistency and/or quality of feedstocks on arrival at a refinery or blending station and/or may be used to provide on-line or at line determination of feedstock quality and property data for input to blending and process refinery optimisation models.

Where the method of the present invention is used at the "well-head" on a drilling site, a number of apparatus' may be operated at different well-heads which use a common transport mechanism, for example a common pipeline, to provide analysis of the crude oil from each well. Analysis of the individual crude oils and appropriate scheduling may allow more optimum composition of the final crude oil blend. In addition, by repeated analysis of the crude oils from different well-heads, changes in the individual crude oils with time can be used to predict the effects on the produced crude oil blend, or influence the blending to maintain a constant quality crude oil blend.

The method may also comprise analysis of the refinery feedstock with one or more further portable analysers, communication of the analysis results to a potential purchaser, and/or combination of the analysis information obtained with a database model as previously described.

Alternatively, or additionally, further analytical tools, such as species-specific sensors, adapted-pH sensors, acoustic sensors, micro-conductivity/capacitance machines, micro-rheological machines can be incorporated into the laboratory-independent device used in the method. Micro-conductivity/capacitance machines and adapted-pH sensors can be used to determine the acidity of a sample, for example. Micro-rheological machines and acoustic sensors can be used to determine the viscosity of a sample, for example. Acoustic sensors also can be used to determine the pour point of a sample, for example.

Preferably the laboratory-independent device includes, or is at least compatible with, remote communications means, such as satellite-based data communication, such that the results of the analyses can be readily communicated to the trader, engineer, or user of the laboratory-independent device.

The assay methods described herein can be carried out at a variety of locations, such as, for example, at a crude oil well, on a crude oil drilling platform where crude oil from multiple wells are being blended, in pipelines that communicate a crude oil (or blends thereof) from one location to another, at the loading/off-loading port of a crude oil vessel/tanker, at an inlet to an oil refinery, or in any intermediate streams in an oil refinery.

### EXAMPLE

This example demonstrates the method of the invention. The NIR spectrum of a crude oil sample using a Bomem FTNIR spectrometer was obtained. NIR spectra were taken in the first overtone region (6300- 5700 wavenumbers), at a path length of 1 mm and a temperature of 40 °C.

The 1 st derivatives of the spectra were used for building the calibration models and for the measurements.

Calibration models for TBP data and density were built using about 40 to 60 calibration samples. These calibration samples were chosen, such that they displayed a variety in crude properties in the desirable ranges. Reference data for the calibration crudes were measured using the corresponding ASTM methods and other appropriate methods as listed in Table 1. Models were built using the partial least squares method ("PLS") method. The models were built using a GRAMS/A1 desktop spectroscopy data processing and management tool available from the Thermo Electron Corporation. Accuracy of the models was verified using an independent set of crude samples.

NIR spectra for the samples were measured using the same parameters. The spectra obtained were than fed into the calibration models and the TBP data and density calculated. Table 2 below sets out the results of the TBP and density as determined by the models.

Additionally, X-ray fluorescence spectroscopy (IP methods 437 and 477) was used to determine the sulphur, nickel, and vanadium contents of the crude sample. This data is also set forth in Table 2 below.

In accordance with the method of the invention the TBP, density, sulfur, nickel, and vanadium data was then correlated using CrudeManager software available from Spiral Software Ltd. to generate a reconstructed determinative assay. Table 3 below sets out the reconstructed assay and compares it with certain actual assay values as determined by conventional assay analytical techniques. As is readily apparent the method of the invention provides a relatively accurate determinative assay of the crude using limited assay information.

CrudeManager is a tool to calculate (reconstruct) a defined set of whole crude properties, properties of individual cuts of a crude oil, or a full crude assay, from a set of key crude data. Typically, this set of data includes TBP data and additional data. The additional data can be, but is not limited to, density, sulphur, nickel, or vanadium. For reconstruction of crude data or of the full crude assay from a limited number of data, CrudeManager has previously been trained with data from a large set of crude assays. It is desirable, that this calibration set cover the variety of crudes, that will be reconstructed.

**Table 2**

| **TBP DATA FROM NIR** | **Yield % wt** |
|---|---|
| Whole Crude | |
| IBP-95 | 8.91 |
| Light naphtha (95 - 149°C) | 8.03 |
| Kerosene (149 - 232°C) | 12.05 |
| Gas Oil (232 - 342 °C) | 19.28 |
| Heavy Gas Oil (342 - 369 °C) | 4.67 |
| Vac. Gas Oil / Waxy Dist. (369 - 509 °C) | 21.64 |
| Vac. Gas Oil/Waxy Dist. (509 - 550 °C) | 5.73 |
| | |
| **Density From NIR** | **g/cc** |
| Whole Crude Density | 0.8652 |
| | |
| **Whole Crude Elemental by XRF** | |
| Sulphur %wt | 1.59 |
| Nickel ppm wt | 18 |
| Vanadium ppm wt | 55 |
| | |
| **Crude Oil Region** | **Russian** |

**Table 3**

| | **Yields %wt** | | **Density g/cc** | | **Sulphur %wt** | |
|---|---|---|---|---|---|---|
| Crude Oil Fractions | **Determined** | Actual | **Determined** | Actual | **Determined** | Actual |
| Whole Crude | | | **0.8652** | 0.867 | **1.58** | 1.59 |
| Gas (<15°C) | **1.9** | 1.5 | **0.5561** | | **0.01** | |
| Gasoline (15-95) | **7.0** | 6.4 | **0.6734** | | **0.02** | |
| Light Naphtha (95-149 °C) | **7.9** | 7.5 | **0.7434** | | **0.03** | |
| Kerosene (149-232 °C) | **12.2** | 13.0 | **0.7936** | | **0.23** | |
| Gas Oil (232 - 342 °C) | **19.2** | 19.3 | **0.8493** | | **0.98** | |
| Heavy Gas Oil (342 - 369 °C) | **4.7** | 4.7 | **0.8831** | | **1.67** | |
| Vac. Gas Oil / Waxy Dist. (369 - 509 °C) | **21.7** | 21.0 | **0.915** | | **1.75** | |
| Vac. Gas Oil / Waxy Dist. (509 - 550 °C) | **5.6** | 5.4 | **0.9447** | | **2.18** | |
| Atmospheric Residue (>369 °C) | **47.1** | 47.9 | **0.9555** | 0.966 | **2.72** | 2.64 |
| Vacuum Residue (>550 °C) | **19.9** | 21.5 | **1.0075** | 1.023 | **3.92** | 3.17 |

| | **Carbon Residue % wt** | | **Nickel ppm** | | **Vanadium ppm** | |
|---|---|---|---|---|---|---|
| Crude Oil Fractions | **Determined** | Actual | **Determined** | Actual | **Determined** | Actual |
| Whole Crude | | | | | | |
| Gas (<15°C) | | | | | | |
| Gasoline (15-95) | | | | | | |
| Light Naphtha (95-149 °C) | | | | | | |
| Kerosene (149-232 °C) | | | | | | |
| Gas Oil (232 - 342 °C) | | | | | | |
| Heavy Gas Oil (342 - 369 °C) | | | | | | |
| Vac. Gas Oil / Waxy Dist. (369 - 509 °C) | | | | | | |
| Vac. Gas Oil / Waxy Dist. (509 - 550 °C) | | | | | | |
| Atmospheric Residue (>369 °C) | **8.4** | 8.7 | **38** | 40 | **117** | 115 |
| Vacuum Residue (>550 °C) | **19.7** | 19.2 | **90** | 89 | **277** | 257 |

The foregoing description is given for clearness of understanding only, and no unnecessary limitations should be understood therefrom, as modifications within the scope of the invention, which is defined solely by the appended claims, may be apparent to those having ordinary skill in the art.

## Claims

1. A method of assaying a hydrocarbon-containing feedstock, the method comprising:
(a) measuring boiling profile and at least one other property of the hydrocarbon containing feedstock with at least two different laboratory-independent techniques wherein each laboratory-independent technique is selected to be predictive of each respective property, wherein at least one of the at least two different laboratory-independent techniques is predictive of the boiling profile and the at least one other is predictive of at least one other property;
(b) transmitting the measurements made in step (a) to a processor capable of reconstructing a determinative assay of the hydrocarbon-containing feedstock from the measurements; and,
(c) reconstructing a determinative assay of the hydrocarbon-containing-feedstock from the measurements, wherein the other property is selected from the group consisting of density, specific gravity, total acidic number, pour point, viscosity, sulfur content, metal content, nitrogen content, and combinations thereof.

2. The method of claim 1, wherein step (a) comprises performing the at least two different laboratory-independent techniques, wherein the at least two laboratory-independent techniques are selected from the group consisting of ultraviolet-visible (UV-Vis) absorbance spectroscopy, infrared (IR) absorbance spectroscopy, UV fluorescence spectroscopy, mid-infrared (MIR) absorbance spectroscopy, near infrared (NIR) absorbance spectroscopy, X-ray fluorescence (XRF) spectroscopy, nuclear magnetic resonance, micro-oscillation, micro-distillation, micro-mass spectrometry, micro-ion mobility spectrometry, and micro-gas chromatography (GC).

3. The method of claim 1, wherein step (a) further comprises correlating data obtained from the at least two different laboratory-independent techniques with the boiling profile and the at least one other property.

4. The method of claim 1, wherein step (a) comprises measuring the boiling profile with at least one of mid-infrared (MIR) absorbance spectroscopy, near infrared (NIR) absorbance spectroscopy, nuclear magnetic resonance, micro-distillation, and micro-gas chromatography (GC).

5. The method of claim 4, wherein step (a) further comprises correlating with the boiling profile of the sample either (i) spectra obtained from the spectroscopy or (ii) data obtained from the micro-distillation, or micro-gas chromatography (GC).

6. The method of claim 1, wherein the at least one other property is selected from the group consisting of density, specific gravity, total acidic number, pour point, viscosity, and combinations thereof, and step (a) comprises measuring the other property by performing at least one technique selected from the group consisting of ultraviolet-visible (UV-Vis) absorbance spectroscopy, infrared (IR) absorbance spectroscopy, UV fluorescence spectroscopy, mid-infrared (MIR) absorbance spectroscopy, near infrared (NIR) absorbance spectroscopy, X-ray fluorescence (XRF) spectroscopy, nuclear magnetic resonance, micro-oscillation, micro-distillation, micro-mass spectrometry, micro-ion mobility spectrometry, and micro-gas chromatography (GC).

7. The method of claim 6, wherein step (a) further comprises correlating data obtained from the at least two different laboratory-independent techniques with the at least one other property.

8. The method of claim 6, wherein the technique is at least one of mid-infrared (MIR) absorbance spectroscopy, near infrared (NIR) absorbance spectroscopy, and nuclear magnetic resonance.

9. The method of claim 1, wherein the other property is selected from the group consisting of sulfur content, metal content, and combinations thereof, and step (a) comprises measuring the property with X-ray fluorescence (XRF) spectroscopy.

10. The method of claim 9, wherein step (a) further comprises correlating data obtained from the X-ray fluorescence (XRF) spectroscopy with the at least one other property.

11. The method of claim 9, wherein the metal is selected from the group consisting of nickel, vanadium, iron, and combinations thereof.

12. The method of claim 1, wherein the at least one other property is metal content and step (a) comprises measuring the at least one other property with ultraviolet-visible (UV-Vis) absorbance spectroscopy.

13. The method of claim 12, wherein step (a) further comprises correlating data obtained from the ultraviolet-visible (UV-Vis) absorbance spectroscopy with the metal content.

14. The method of claim 12, wherein the metal is selected from the group consisting of nickel, vanadium, iron, and combinations thereof.

15. The method of claim 1, wherein the boiling profile is the true boiling profile.

## Patentansprüche

1. Verfahren zur Prüfung eines kohlenwasserstoffhaltigen Rohmaterials, wobei das Verfahren Folgendes umfasst:
(a) Messen des Siedeprofils und mindestens einer anderen Eigenschaft des kohlenwasserstoffhaltigen Rohmaterials mit mindestens zwei verschiedenen laborunabhängigen Techniken, wobei jede laborunabhängige Technik dergestalt ausgewählt ist, dass sie jede jeweilige Eigenschaft anzeigt, wobei mindestens eine der mindestens zwei verschiedenen laborunabhängigen Techniken das Siedeprofil anzeigt und die mindestens eine andere mindestens eine andere Eigenschaft anzeigt;
(b) Übertragen der in Schritt (a) vorgenommenen Messungen an einen Prozessor, der zum Rekonstruieren einer maßgebenden Prüfung des kohlenwasserstoffhaltigen Rohmaterials anhand der Messungen fähig ist; und
(c) Rekonstruieren einer maßgebenden Prüfung des kohlenwasserstoffhaltigen Rohmaterials anhand der Messungen, wobei die andere Eigenschaft aus der Gruppe ausgewählt ist, bestehend aus der Dichte, dem spezifischen Gewicht, der Gesamtsäurezahl, dem Stockpunkt, der Viskosität, dem Schwefelgehalt, dem Metallgehalt, dem Stickstoffgehalt und Kombinationen davon.

2. Verfahren nach Anspruch 1, wobei Schritt (a) das Durchführen der mindestens zwei verschiedenen laborunabhängigen Techniken umfasst, wobei die mindestens zwei laborunabhängigen Techniken aus der Gruppe ausgewählt sind, bestehend aus der Absorptionsspektroskopie im ultraviolett-sichtbaren Bereich (UV-Vis), der Infrarot(IR)-Absorptionsspektroskopie, der UV-Fluoreszenzspektroskopie, der Absorptionsspektroskopie im mittleren Infrarotbereich (MIR), der Absorptionsspektroskopie im nahen Infrarotbereich (NIR), der Röntgenfluoreszenzspektroskopie (XRF-Spektroskopie), der magnetischen Kernresonanz, der Mikrooszillation, der Mikrodestillation, der Mikromassenspektrometrie, der Mikro-Ionenmobilitätsspektrometrie und der Mikro-Gaschromatographie (GC).

3. Verfahren nach Anspruch 1, wobei Schritt (a) weiter das Korrelieren der anhand der mindestens zwei verschiedenen laborunabhängigen Techniken, mit dem Siedeprofil erhaltenen Daten und der mindestens einen anderen Eigenschaft umfasst.

4. Verfahren nach Anspruch 1, wobei Schritt (a) das Messen des Siedeprofils mit mindestens einer von der Absorptionsspektroskopie im mittleren Infrarotbereich (MIR), der Absorptionsspektroskopie im nahen Infrarotbereich (NIR), der magnetischen Kernresonanz, der Mikrodestillation und der Mikro-Gaschromatographie (GC) umfasst.

5. Verfahren nach Anspruch 4, wobei Schritt (a) weiter das Korrelieren mit dem Siedeprofil der Probe von entweder (i) anhand der Spektroskopie erhaltenen Spektren oder (ii) anhand der Mikrodestillation oder der Mikro-Gaschromatographie (GC) erhaltenen Daten umfasst.

6. Verfahren nach Anspruch 1, wobei die mindestens eine andere Eigenschaft aus der Gruppe ausgewählt ist, bestehend aus der Dichte, dem spezifischen Gewicht, der Gesamtsäurezahl, dem Stockpunkt, der Viskosität und Kombinationen davon und Schritt (a) das Messen der anderen Eigenschaft mittels Durchführen mindestens einer Technik umfasst, die aus der Gruppe ausgewählt ist, bestehend aus der Absorptionsspektroskopie im ultraviolett-sichtbaren Bereich (UV-Vis), der Infrarot(IR)-Absorptionsspektroskopie, der UV-Fluoreszenzspektroskopie, der Absorptionsspektroskopie im mittleren Infrarotbereich (MIR), der Absorptionsspektroskopie im nahen Infrarotbereich (NIR), der Röntgenfluoreszenzspektroskopie (XRF-Spektroskopie), der magnetischen Kernresonanz, der Mikrooszillation, der Mikrodestillation, der Mikromassenspektrometrie, der Mikro-Ionenmobilitätsspektrometrie und der Mikro-Gaschromatographie (GC).

7. Verfahren nach Anspruch 6, wobei Schritt (a) weiter das Korrelieren der anhand der mindestens zwei verschiedenen laborunabhängigen Techniken erhaltenen Daten mit der mindestens einen anderen Eigenschaft umfasst.

8. Verfahren nach Anspruch 6, wobei die Technik mindestens eine von der Absorptionsspektroskopie im mittleren Infrarotbereich (MIR), der Absorptionsspektroskopie im nahen Infrarotbereich (NIR) und der magnetischen Kernresonanz ist.

9. Verfahren nach Anspruch 1, wobei die andere Eigenschaft aus der Gruppe ausgewählt ist, bestehend aus dem Schwefelgehalt, dem Metallgehalt und Kombinationen davon und Schritt (a) das Messen der Eigenschaft mittels der Röntgenfluoreszenzspektroskopie (XRF-Spektroskopie) umfasst.

10. Verfahren nach Anspruch 9, wobei Schritt (a) weiter das Korrelieren der anhand der Röntgenfluoreszenzspektroskopie (XRF-Spektroskopie) erhaltenen Daten mit der mindestens einen anderen Eigenschaft umfasst.

11. Verfahren nach Anspruch 9, wobei das Metall aus der Gruppe ausgewählt ist, bestehend aus Nickel, Vanadium, Eisen und Kombinationen davon.

12. Verfahren nach Anspruch 1, wobei die mindestens eine andere Eigenschaft der Metallgehalt ist und Schritt (a) das Messen der mindestens einen anderen Eigenschaft mit der Absorptionsspektroskopie im ultraviolett-sichtbaren Bereich (UV-Vis) umfasst.

13. Verfahren nach Anspruch 12, wobei Schritt (a) weiter das Korrelieren der anhand der Absorptionsspektroskopie im ultraviolett-sichtbaren Bereich (UV-Vis) erhaltenen Daten mit dem Metallgehalt umfasst.

14. Verfahren nach Anspruch 12, wobei das Metall aus der Gruppe ausgewählt ist, bestehend aus Nickel, Vanadium, Eisen und Kombinationen davon.

15. Verfahren nach Anspruch 1, wobei das Siedeprofil das wahre Siedeprofil ist.

## Revendications

1. Procédé d'analyse d'une charge d'alimentation contenant un hydrocarbure, le procédé comprenant :
(a) la mesure du profil d'ébullition et d'au moins une autre propriété de la charge d'alimentation contenant un hydrocarbure par au moins deux différentes techniques indépendantes du laboratoire, chaque technique indépendante du laboratoire étant sélectionnée pour être prédictive de chaque propriété respective, au moins l'une desdites au moins deux différentes techniques indépendantes du laboratoire étant prédictive du profil d'ébullition et ladite au moins une autre étant prédictive d'au moins une autre propriété ;
(b) la transmission des mesures réalisées à l'étape (a) à un processeur capable de reconstruire une analyse déterminative de la charge d'alimentation contenant un hydrocarbure à partir des mesures ; et
(c) la reconstruction d'une analyse déterminative de la charge d'alimentation contenant un hydrocarbure à partir des mesures, l'autre propriété étant sélectionnée dans le groupe constitué de la densité, du poids spécifique, de l'indice d'acide total, du point d'écoulement, de la viscosité, de la teneur en soufre, de la teneur en métal, de la teneur en azote, et de combinaisons de ceux-ci.

2. Procédé selon la revendication 1, dans lequel l'étape (a) comprend la mise en oeuvre desdites au moins deux différentes techniques indépendantes du laboratoire, dans lequel lesdites au moins deux techniques indépendantes du laboratoire sont sélectionnées dans le groupe constitué de la spectroscopie d'absorption dans le rayonnement ultraviolet-visible (UV-Vis), de la spectroscopie d'absorption dans le rayonnement infrarouge (IR), de la spectroscopie de fluorescence dans le rayonnement UV, de la spectroscopie d'absorption dans le rayonnement infrarouge moyen (MIR), de la spectroscopie d'absorption dans le rayonnement infrarouge proche (NIR), de la spectroscopie de fluorescence des rayons X (XRF), de la résonance magnétique nucléaire, de la micro-oscillation, de la micro-distillation, de la micro-spectrométrie de masse, de la micro-spectrométrie de mobilité ionique, et de la micro-chromatographie en phase gazeuse (CG).

3. Procédé selon la revendication 1, dans lequel l'étape (a) comprend en outre la mise en corrélation de données obtenues à partir desdites au moins deux différentes techniques indépendantes du laboratoire avec le profil d'ébullition et ladite au moins une autre propriété.

4. Procédé selon la revendication 1, dans lequel l'étape (a) comprend la mesure du profil d'ébullition par au moins l'une de la spectroscopie d'absorption dans le rayonnement infrarouge moyen (MIR), de la spectroscopie d'absorption dans le rayonnement infrarouge proche (NIR), de la résonance magnétique nucléaire, de la micro-distillation, et de la micro-chromatographie en phase gazeuse (CG).

5. Procédé selon la revendication 4, dans lequel l'étape (a) comprend en outre la mise en corrélation avec le profil d'ébullition de l'échantillon soit (i) de spectres obtenus par la spectroscopie, soit (ii) de données obtenues par la micro-distillation, ou la micro-chromatographie en phase gazeuse (CG).

6. Procédé selon la revendication 1, dans lequel ladite au moins une autre propriété est sélectionnée dans le groupe constitué de la densité, du poids spécifique, de l'indice d'acide total, du point d'écoulement, de la viscosité, et de combinaisons de ceux-ci, et l'étape (a) comprend la mesure de l'autre propriété par mise en oeuvre d'au moins une technique sélectionnée dans le groupe constitué de la spectroscopie d'absorption dans le rayonnement ultraviolet-visible (UV-Vis), de la spectroscopie d'absorption dans le rayonnement infrarouge (IR), de la spectroscopie de fluorescence dans le rayonnement UV, de la spectroscopie d'absorption dans le rayonnement infrarouge moyen (MIR), de la spectroscopie d'absorption dans le rayonnement infrarouge proche (NIR), de la spectroscopie de fluorescence des rayons X (XRF), de la résonance magnétique nucléaire, de la micro-oscillation, de la micro-distillation, de la micro-spectrométrie de masse, de la micro-spectrométrie de mobilité ionique, et de la micro-chromatographie en phase gazeuse (CG).

7. Procédé selon la revendication 6, dans lequel l'étape (a) comprend en outre la mise en corrélation de données obtenues à partir desdites au moins deux différentes techniques indépendantes du laboratoire avec ladite au moins une autre propriété.

8. Procédé selon la revendication 6, dans lequel la technique est l'une au moins de la spectroscopie d'absorption dans le rayonnement infrarouge moyen (MIR), de la spectroscopie d'absorption dans le rayonnement infrarouge proche (NIR), et de la résonance magnétique nucléaire.

9. Procédé selon la revendication 1, dans lequel l'autre propriété est sélectionnée dans le groupe constitué de la teneur en soufre, de la teneur en métal, et de combinaisons de celles-ci, et l'étape (a) comprend la mesure de la propriété par spectroscopie de fluorescence des rayons X.

10. Procédé selon la revendication 9, dans lequel l'étape (a) comprend en outre la mise en corrélation de données obtenues par la spectroscopie de fluorescence des rayons X avec ladite au moins une autre propriété.

11. Procédé selon la revendication 9, dans lequel le métal est sélectionné dans le groupe constitué du nickel, du vanadium, du fer, et de combinaisons de ceux-ci.

12. Procédé selon la revendication 1, dans lequel ladite au moins une autre propriété est la teneur en métal et l'étape (a) comprend la mesure de ladite au moins une autre propriété par spectroscopie d'absorption dans le rayonnement ultraviolet-visible (UV-Vis).

13. Procédé selon la revendication 12, dans lequel l'étape (a) comprend en outre la mise en corrélation de données obtenues par la spectroscopie d'absorption dans le rayonnement ultraviolet-visible (UV-Vis) avec la teneur en métal.

14. Procédé selon la revendication 12, dans lequel le métal est sélectionné dans le groupe constitué du nickel, du vanadium, du fer, et de combinaisons de ceux-ci.

15. Procédé selon la revendication 1, dans lequel le profil d'ébullition est le vrai profil d'ébullition.
